# EUROPÄISCHE PATENTSCHRIFT

(11) **EP 1 117 641 B1**
(45) Veröffentlichungstag und Bekanntmachung des Hinweises auf die Patenterteilung: **24.03.2004**
(21) Anmeldenummer: 99970081.8
(22) Anmeldetag: 21.09.1999
(51) Int. Cl.: C07D 209/12, A61K 7/42, A61K 31/40, C07D 209/24, C07D 209/14, C07D 403/04

(54) **INDOLINDERIVATE ALS LICHTSCHUTZMITTEL**
INDOLIN DERIVATIVES AS SUN PROTECTION AGENTS
DERIVES D'INDOLINE UTILISES COMME AGENTS PHOTOPROTECTEURS

(30) Priorität: 02.10.1998 EP 98810993
(43) Veröffentlichungstag der Anmeldung: 25.07.2001
(73) Patentinhaber: Ciba Specialty Chemicals Holding Inc., 4057 Basel (CH)
(72) Erfinder: ZINK, Rudolf, CH-4106 Therwil (CH); LUTHER, Helmut, D-79639 Grenzach-Wyhlen (DE)
(86) Internationale Anmeldenummer: PCT/EP1999/006984
(87) Internationale Veröffentlichungsnummer: WO 2000/020388

(56) Entgegenhaltungen:
- DE-A- 2 152 948
- DE-A- 19 539 623
- GB-A- 929 394
- US-A- 4 522 808
- US-A- 4 889 410
- CHEMICAL ABSTRACTS, vol. 121, no. 16, 17. Oktober 1994 (1994-10-17) Columbus, Ohio, US; abstract no. 191470c, ISHIOKA,TAKAYUKI ET AL: "Short wavelenght laser-sensitive high-density optical recording medium." XP002089201 -& DATABASE CHEMICAL ABSTRACTS [Online] XP002090518 & JP 05 305773 A (NIPPON COLUMBIA)
- CHEMICAL ABSTRACTS, vol. 115, no. 20, 18. November 1991 (1991-11-18) Columbus, Ohio, US; abstract no. 210209u, IMAKOMI, HIROSHI ET AL: "Yellow cationic dyes and their use." XP002089202 -& DATABASE CHEMICAL ABSTRACTS [Online] 115:210209, XP002090519 & JP 03 134063 A (HODOGAYA CHEMICAL CO.,LTD.)
- CHEMICAL ABSTRACTS, vol. 118, no. 25, 21. Juni 1993 (1993-06-21) Columbus, Ohio, US; abstract no. 255031h, RATOVSKII, G.V. ET AL: "Intramolecular interactions in phosphorus-substituted 1,3,3-trimethyl-2-methyleneindolines." XP002089203 -& DATABASE CHEMICAL ABSTRACTS [Online] 118:255031, XP002090520 & ZH. OBSHCH. KHIM., Bd. 62, Nr. 9, - 1992 Seiten 2046-2051,
- CHEMICAL ABSTRACTS, vol. 97, no. 10, 6. September 1982 (1982-09-06) Columbus, Ohio, US; abstract no. 73922q, "Methine dyes." XP002089204 -& DATABASE CHEMICAL ABSTRACTS [Online] 97:73922, XP002090521 & JP 82 030760 A (SUMITOMO CHEMICAL COMPANY, LTD.)

## Beschreibung

Die vorliegende Erfindung betrifft Indolinderivate sowie die Verwendung dieser Verbindungen als Lichtschutzmittel.

Indolinverbindungen sind aus der Farbstoffchemie bekannt und dienen dort als Zwischenoder Ausgangsprodukte. Indoxyl z.B. tritt als Zwischenprodukt bei der technischen indigosynthese auf. Trimethyl-2-methylenindolin (Fischer-Base) dient als Ausgangsverbindung zur Herstellung von Polymethinfarbstoffen.

In C.A. 121(16) (1994) werden Indolinderivate offenbart, die als optische Aufzeichnungsmaterialien dienen.

Die GB-0,929,394 offenbart ein Verfahren zum Färben oder Drucken von synthetischen polymeren Verbindungen mit Farbstoffen aus der Klasse der Indolinverbindungen.

Die US-A-4,889,410 offenbart Indolinverbindungen, die als Farbfilter eingesetzt werden.

C.A. 115(20) (1991) offenbart Idolinderivate, die.als gelbe kationische Farbstoffe Verwendung finden.

C.A. 97(10) (1982) offenbart Indolinderivate, die zum Färben von Polyesterfasern verwendet werden.

De-2152948 offenbart ein Verfahren zum Färben von anionisch modifizierten synthetischen Farbstoffen mit Indolinderivaten.

Die DE-19539623 offenbart spezifische Sulfonsäuren, die Verwendung als UV-Absorber, insbesondere in Sonnenschutzmitteln Verwendung finden.

Die US-A-4,522,808 offenbart 2-Phenyl-Indol als UV-Absorber, die in Mitteln gegen Sonnenbrand Verwendung finden.

Überraschenderweise wurde gefunden, dass gewisse Indolinderivate sich auch als Lichtschutzmittel eignen.

Gegenstand der vorliegenden Erfindung ist daher die Verwendung von Indolinderivaten der Formel 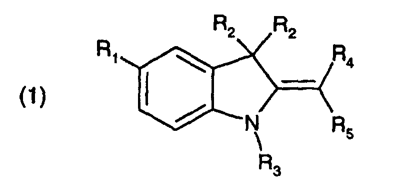 worin
- R₁: Wasserstoff; C₁-C₅-Alkyl; C₁-C₁₈-Alkoxy; oder Halogen;
- R₂: C₁-C₈-Alkyl; C₅-C₇-Cycloalkyl; C₆-C₁₀-Aryl;
- R₃: C₁-C₁₈-Alkyl; oder ein Rest der Formel (1a) 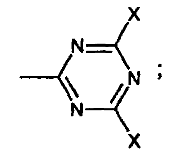
- R₄: Wasserstoff; oder ein Rest der Formel (1b) 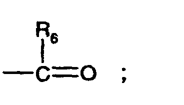
- R₅: einen Rest der Formel (1c) 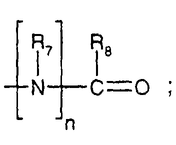 C₁-C₁₈-Alkoxy; oder einen Rest der Formel (1d) 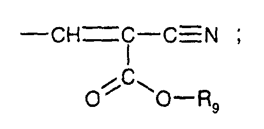
- R₆ und R₇: unabhängig voneinander Wasserstoff; oder C₁-C₅-Alkyl;
- R₈: Wasserstoff; C₁-C₅-Alkyl; C₅-C₇-Cycloalkyl; Phenyl; Phenyl-C₁-C₃-Alkyl;
- R₉: C₁-C₁₈-Alkyl;
- X: Hal; ein Rest der Formel (1e) 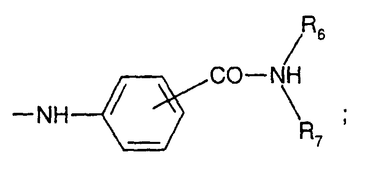 oder(1f) 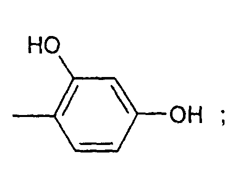 und
- n: 0; oder 1;
bedeuten, als Lichtschutzmittel.

C₁-C₁₈-Alkyl sind geradkettige oder verzweigte Alkylreste wie z.B. Methyl, Ethyl, n-Propyl, Isopropyl, n-Butyl, sek.Butyl, tert.Butyl, Amyl, Isoamyl oder tert.Amyl, Hexyl, Heptyl, Octyl, Isooctyl, Nonyl, Decyl, Undecyl, Dodecyl, Tetradecyl, Pentadecyl, Hexadecyl, Heptadecyl oder Octadecyl.

C₁-C₁₈-Alkoxy bedeutet z.B. Methoxy, Ethoxy, n-Propoxy, Isopropoxy, n-Butoxy, sek.Butoxy, tert.Butoxy, Amyloxy, Isoamyloxy oder tert.Amyloxy, Hexyloxy, Heptyloxy, Octyloxy, Isooctyloxy, Nonyloxy, Decyloxy, Undecyloxy, Dodecyloxy, Tetradecyloxy, Pentadecyloxy, Hexadecyloxy, Heptadecyloxy oder Octadecyloxy.

Halogen bedeutet Fluor oder Brom, und insbesondere Chlor.

C₅-C₇-Cycloalkyl bedeutet z.B. Cyclopentyl, Cycloheptyl und insbesondere Cyclohexyl.

C₆-C₁₀-Aryl bedeutet Phenyl oder Naphthyl.

Vorzugsweise werden erfindungsgemäss Verbindungen der Formel (1) verwendet, worin R₅ einen Rest der Formel (1g) 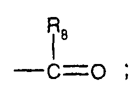 und R₈ Wasserstoff; C₁-C₅-Alkyl; oder Phenyl, insbesondere Wasserstoff oder Methyl bedeuten.

Weiterhin sind Verbindungen der Formel (1) interessant, worin R₅ C₁-C₁₈-Alkoxy, und insbesondere einen Rest der Formel (1h) 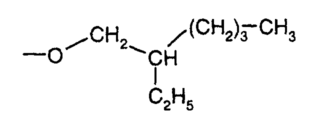 bedeutet.

Weiterhin sind Verbindungen der Formel (1) interessant, worin R₅ einen Rest der Formel (1I) 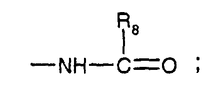 und R₈ Wasserstoff; oder Methyl bedeutet.

Im Vordergrund des Interesses stehen auch Verbindungen der Formel (1), worin R₃ C₃-C₁₈iso-Alkyl bedeutet.

Beispielhafte erfindungsgemäss eingesetzte Verbindungen entsprechen den Formeln

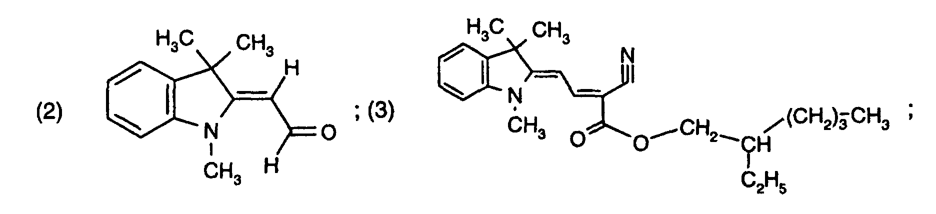

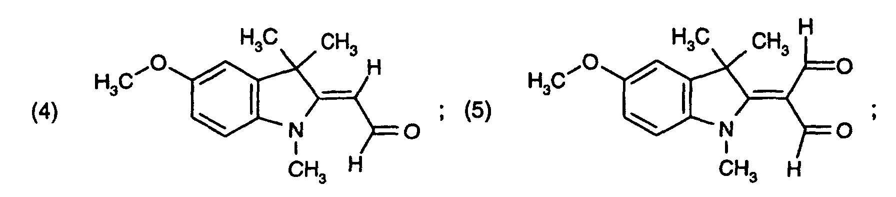

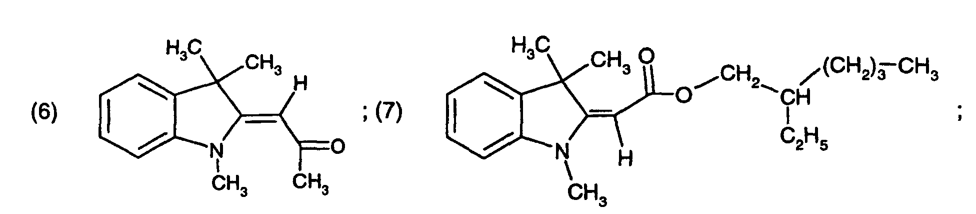

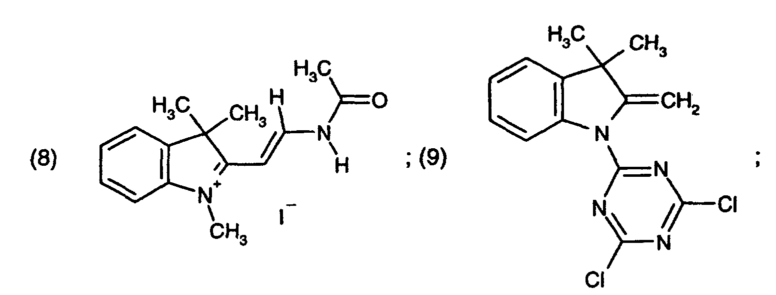

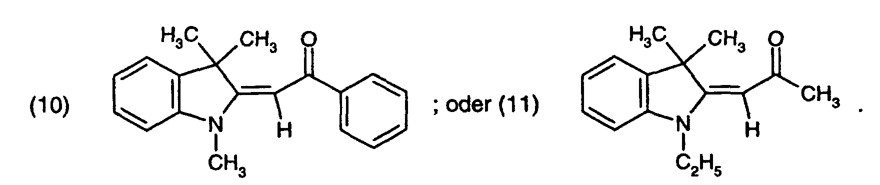

Bei den Verbindungen der Formeln (1) bis (11) handelt es sich z.T. um bekannte Verbindungen.

Die Herstellung der erfindungsgemäss verwendeten Indolinderivate erfolgt in an sich bekannter Weise durch Kondensation der Indolinverbindung (Fischer-Base) der Formel 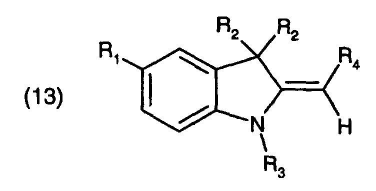 mit dem entsprechenden Säurechlorid oder einer CH-aciden Verbindung zur Verbindung der Formel (1).

Die Umsetzung erfolgt gewöhnlich bei Temperaturen von 20 bis 110°C in Gegenwart eines inerten Lösungsmittels, wie z.B. Petrolether, Toluol, Xylol, Xylol-Isomerengemisches oder eines halogenierten Kohlenwasserstoffes, wie z.B. CH₂Cl₂. Die Reaktion kann aber auch ohne Lösungsmittel durchgeführt werden. Die Reaktionszeit liegt gewöhnlich bei wenigen Minuten und kann bis zu einigen Stunden dauern.

Die Verbindungen der Formel (1) eignen sich insbesondere als UV-Filter, d.h. zum Schützen von ultraviolett empfindlichen organischen Materialien, insbesondere der Haut und Haare von Menschen und Tieren vor der schädigenden Einwirkung von UV-Strahlung. Diese Verbindungen eignen sich daher als nicht-therapeutische Lichtschutzmittel in kosmetischen, pharmazeutischen und veterinärmedizinischen Zusammensetzungen. Diese Verbindungen können sowohl gelöst als auch im mikronisierten Zustand verwendet werden.

Ein weiterer Erfindungsgegenstand bildet eine kosmetische Zusammensetzung, enthaltend eine Verbindung der Formel (1).

Für die kosmetische Verwendung haben diese Lichtschutzmittel - sofern sie nicht wasserlöslich sind - gewöhnlich eine mittlere Partikelgrösse im Bereich von 0,02 bis 2, vorzugsweise 0,05 bis 1,5, und ganz besonders von 0,1 bis 1,0 m. Die unlöslichen erfindungsgemässen Lichtschutzmittel können durch übliche Methoden, z.B. Mahlen mit z.B. einer Düsen-, Kugel-, Vibrations- oder Hammermühle auf die gewünschte Partikelgrösse gebracht werden. Vorzugsweise wird das Mahlen in Anwesenheit von 0,1 bis 30, vorzugsweise 0,5 bis 15 Gew.-%, bezogen auf den UV-Absorber, einer Mahlhilfe wie z.B. eines alkylierten Vinylpyrrolidon-Polymers, eines Vinylpyrrolidon-Vinylacetat-Copolymers, eines Acylglutamates, eines Alkylpolyglucosides oder insbesondere eines Phospholipids durchgeführt.

Die Lichtschutzmittel können auch trocken in Pulverform verwendet werden. Dazu werden die Lichtschutzmittel bekannten Mahlverfahren unterzogen wie Vakuumzerstäubung, Gegenstromsprühtrocknung usw.. Diese Pulver haben eine Partikelgrösse von 0,1 nm bis 2 µm. Zur Vermeidung von Agglomerationsprozessen können die Lichtschutzmittel vor dem Pulverisierungsprozess mit einer oberflächenaktiven Verbindung wie z.B. einem anionischen, nichtionogenen, oder amphoteren Tensid, wie z.B. mit Phospholipiden oder bekannten Polymeren, wie PVP, Acrylaten usw. überzogen werden.

Die kosmetische Zusammensetzung kann neben dem erfindungsgemässen UV-Absorber auch noch einen oder mehrere weitere UV-Schutzstoffe der folgenden Substanzklassen enthalten:
1. p-Aminobenzoesäurederivate, wie z.B. 4-Dimethylaminobenzoesäure-2-ethylhexylester;
2. Salicylsäurederivate, wie z.B. Salicylsäure-2-ethylhexylester;
3. Benzophenonderivate, wie z.B. 2-Hydroxy-4-methoxybenzophenon und sein 5-sulfonsäurederivat;
4. Dibenzoylmethanderivate, wie z.B. 1-(4-tert.-Butylphenyl)-3-(4-methoxyphenyl)propan-1,3-dion;
5. Diphenylacrylate, wie z.B. 2-Ethylhexyl-2-cyano-3,3-diphenyl acrylat und 3-(Benzofuranyl)-2-cyanoacrylat;
6. 3-Imidazol-4-yl-acrylsäure und -ester;
7. Benzofuranderivate, insbesondere 2-(p-Aminophenyl)benzofuranderivate, beschrieben in der EP-A-582,189, US-A-5,338,539, US-A-5,518,713 und der EP-A-613,893;
8. polymere UV-Absorber wie z.B. die in der EP-A-709,080 beschriebenen Benzylidenmalonatderivate;
9. Zimtsäurederivate, wie z.B. die in der US-A-5,601,811 und WO 97/00851 offenbarten 4-Methoxyzimtsäure-2-ethylhexylester bzw. Isoamylester oder Zimtsäurederivate;
10. Campherderivate, wie z.B. 3-(4'-Methyl)benzyliden-bornan-2-on, 3-Benzyliden-bornan-2-on, N-[2(und 4)-2-Oxyborn-3-yliden-methyl)-benzyl]acrylamid-Polymer, 3-(4'-Trimethylammonium)-benzyliden-bornan-2-on methylsulfat, 3,3'-(1,4-Phenylendimethin)-bis(7,7-dimethyl-2-oxo-bicyclo-[2.2.1]heptan-1-methansulphonsäure) und Salze, 3-(4'-Sulfo)benzyliden-bornan-2-on und Salze;
11. Trianilino-s-Triazinderivate, wie z.B. 2,4,6-Trianilin-(p-carbo-2'-ethyl-1'-oxi)-1,3,5-triazin sowie die in der US-A-5,332,568, EP-A-517,104, EP-A-507,691, WO 93/17002 und EP-A-570,838 offenbarten UV-Absorber;
12. 2-Hydroxyphenyl-Benzotriazol-Derivate;
13. 2-Phenylbenzimidazol-5-sulfonsäure und deren Salze;
14. Menthyl-o-aminobenzoat;
15. anorganische Mikropigmente, wie z.B. TiO₂ (unterschiedlich umhüllt);
16. N-substituierte Benzimidazole, wie in der EP-A-0,843,995 beschrieben;
17. Hydroxyphenylbenztriazole und Derivate, insbesonder Siloxan-Derivate;
18. Siloxane von Oxanilid-Derivaten, wei in der EP-A-0,712,856 beschrieben.

Auch die in "Sunscreens", Eds. N.J. Lowe, N.A.Shaath, Marcel Dekker, Inc. , New York and Basel oder in Cosmetics & Toiletries (107), 50ff (1992) beschriebenen UV-Absorber können als zusätzliche UV-Schutzstoffe in der erfindungsgemässen Formulierung verwendet werden.

Weiterhin kann die erfindungsgemässe kosmetische Zusammensetzung auch zusammen mit bekannten Antioxidantien, wie z.B. Aminosäuren (z.B. Glycerin, Histidin, Tyrosin, Tryptophan) und deren Derivate, Peptiden (z.B. Camosin) und deren Derivaten, Vitamin E bzw. Vitamin A und deren Derivaten, Derivaten des Vitamin C, Carotinoiden, Flavanoiden und deren Derivaten und Ubichinonen oder HALS (="Hindered Amine Light Stabilizers")-Verbindungen eingesetzt werden.

Die kosmetischen Zusammensetzungen enthalten 0,1 bis 15, vorzugsweise 0,5 bis 10 Gew.-%, bezogen auf das Gesamtgewicht der Zusammensetzung, eines Lichtschutzmittels der Formel (1) oder eines Gemisches aus Lichtschutzmitteln und einen kosmetisch verträglichen Hilfsstoff.

Die Herstellung der kosmetischen Zusammensetzungen kann durch physikalisches Mischen des oder der UV-Absorber mit dem Hilfsstoff durch gewöhnliche Methoden, wie z.B. durch einfaches Zusammenrühren der Einzelkomponenten, insbesondere durch Nutzung der Lösungseigenschaften von bereits bekannten kosmetischen UV-Absorbem wie z.B OMC (Octyl-Methyl-Cinnamat), Salicylsäure-isooctylester u.a., erfolgen.

Die kosmetischen Zusammensetzungen können als Wasser-in-Öl- oder Öl-in-Wasser-Emulsion, als Öl-in-Alkohol-Lotion, als vesikulare Dispersion eines ionischen oder nichtionischen amphiphilen Lipids, als Gel, fester Stift oder als Aerosol-Formulierung formuliert werden.

Als Wasser-in-Öl- oder Öl-in-Wasser-Emulsion enthält der kosmetisch verträgliche Hilfsstoff vorzugsweise 5 bis 50% einer Ölphase, 5 bis 20% eines Emulgators und 30 bis 90% Wasser. Die Ölphase kann dabei irgendein für kosmetische Formulierungen geeignetes Öl enthalten, wie z.B. ein oder mehrere Kohlenwasserstofföle, ein Wachs, ein natürliches Öl, ein Silikonöl, einen Fettsäureester oder einen Fettalkohol. Bevorzugte Mono- oder Polyole sind Ethanol, Isopropanol, Propylenglykol, Hexylenglycol, Glycerin und Sorbitol. Es können auch zwei- oder/und dreiwertige Metallsalze (Erdalkali, Al³⁺ u.a.) einer oder mehrerer Alkylcarbonsäuren verwendet werden.

Für die kosmetischen Zusammensetzungen können jede konventionell einsetzbaren Emulgatoren verwendet werden, wie z.B. einer oder mehrere ethoxylierte Ester von natürlichen Derivaten, wie z.B. polyethoxylierte Ester von hydrogeniertem Castor-Öl; oder Silikonöl-Emulgatoren wie z.B. Silikonpolyol; gegebenenfalls ethoxylierte Fettsäureseifen; Fettalkohole oder Fettsäuren sowie deren Polyoxethylenderivate; gegebenenfalls ethoxylierte Sorbitanester; ethoxylierte Fettsäuren oder Fettsäureester; oder ethoxylierte Glyceride.

Weitere geeignete Emulgatoren sind partielle Fettsäureester mehrwertiger Alkohole wie Glykol, 1,2-Propylenglykol, Glycerin, Sorbit und Pentaerythrit, ebenso Eiweiss-Fettsäure-Kondensationsprodukte und Lanolinderivate, Salze von Alkylcarbonsäuren, Alkylsulfaten oder -sulfonaten oder Polyglykolethem. Weiterhin können auch Mischungen von anionaktiven und nichtionogenen Emulgatoren oder Gemische aus rein nichtionischen grenzflächenaktiven Substanzen mit verschiedenen HLB (Hydrophilic-Lipophilic Balance)-Werten eingesetzt werden. Auch Mischungen von Fettalkohol- und Fettsäurepolyglykolethern oder oxethylierten Fetten sind gebräuchlich.

Die kosmetischen Zusammensetzungen können auch weitere Komponenten, wie z.B. Emollients, Emulsionsstabilisatoren, Haut-Feuchthaltemittel, Hautbräunungsbeschleuniger, Verdickungsmittel wie z.B. Xanthan, Feuchtigkeit-Retentionsmittel wie z.B. Glycerin, Konservierungsmittel, Duft- und Farbstoffe enthalten.

Erfindungsgemässe kosmetische Formulierungen beinhalten verschiedene kosmetische Mittel. Insbesondere kommen z.B. die folgenden Mittel in Betracht:
- Mittel zur Hautpflege, wie z.B. Hautwasch- und Reinigungsmittel in Form von stückförmigen oder flüssigen Seifen, Syndets oder Waschpasten,
- Badepräparate, wie z.B. flüssige (Schaumbäder, Milche, Duschpräparate) oder feste Badepräparate, wie z.B. Badetabletten und Badesalze;
- Hautpflegemittel, wie z.B. Hautemulsionen, Mehrfachemulsionen oder Hautöle;
- Dekorative Körperpflegemittel, wie z.B. Gesichts-Make-ups in Form von Tages- oder Pudercremes, Gesichtspuder (lose und gepresst), Rouge oder Creme-Make-ups, Augenpflegemittel, wie z.B. Lidschattenpräparate, Wimperntusche, Eyeliner, Augencremes oder Eye-Fix-Cremes; Lippenpflegemittel, wie z.B. Lippenstift, Lip Gloss, Lippenkonturstift, Nagelpflegemittel, wie Nagellack, Nagellackentferner, Nagelhärter, oder Nagelhautentferner;
- Intimpflegemittel, wie z.B. Intim-Waschlotionen oder Intimsprays;
- Fusspflegemittel, wie z.B. Fussbäder, Fusspuder, Fusscremes bzw. Fussbalsame, spezielle Deomittel und Antitranspirantien oder hornhautbeseitigende Mittel;
- Lichtschutzmittel, wie Sonnenmilche, -lotionen, -cremes, -öle, Sun-blockers oder Tropicals, Vorbräunungspräparate oder After-sun-Präparate;
- Hautbräunungsmittel, wie z.B. Selbstbräunungscremes;
- Depigmentierungsmittel, wie z.B. Präparate zur Hautbleichung oder Mittel zur Hautaufhellung;
- Insektenabweisende Mittel ("Repellents"), wie z.B. Insektenöle, -lotionen, -sprays, oder
- stifte;
- Deodorantien, wie Deosprays, Pumpsprays, Deogele, -stifte oder -roller;
- Antitranspirantien, wie z.B. Antitranspirantstifte, -cremes oder -roller;
- Mittel zur Reinigung und Pflege von unreiner Haut, wie z.B. Syndets (fest oder flüssig), Peeling- oder Scrubb-Präparate oder Peeling-Masken;
- Haarentfernungsmittel in chemischer Form (Depilation), wie z.B. Haarentfernungspulver, flüssige Enthaarungsmittel, cremige oder pastöse Enthaarungsmittel, Enthaarungsmittel in Gelform oder Aerosolschäume;
- Rasiermittel, wie z.B. Rasierseife, schäumende Rasiercremes, nichtschäumende Rasiercremes, -schäume, -gele, Preshave-Präparate für die Trockenrasur, Aftershaves oder Aftershave-Lotionen;
- Duftmittel, wie z.B. Duftwässer (Eau de Cologne, Eau de Toilette, Eau de Parfum, Parfum de Toilette, Parfüm), Parfümöle oder Parfümcremes;
- Mittel zur Zahn-, Zahnersatz- und Mundpflege, wie z.B. Zahncremes, Gel-Zahnoremes, Zahnpulver, Mundwasserkonzentrate, Anti-Plaque-Mundspülungen, Prothesenreiniger oder Prothesenhaftmittel;
- Kosmetische Mittel zur Haarbehandlung, wie z.B. Haarwaschmittel in Form von Schampoos, Haarkonditioniermittel, Haarpflegemittel, wie z.B. Vorbehandlungsmittel, Haarwasser, Frisiercremes, Frisiergele, Pomaden, Haarspülungen, Kurpackungen, Intensivhaarkuren, Mittel zur Haarverformung, wie z.B. Wellmittel zur Herstellung von Dauerwellen (Heisswelle, Mildwelle, Kaltwelle), Haarglättungspräparate, flüssige Haarfestiger, Haarschäume, Haarsprays, Blondiermittel, wie. z.B. Wasserstoffperoxidlösungen, aufhellende Schampoos, Blondiercremes, Blondierpulver, Blondierbreie oder -öle, temporäre, semitemporäre oder permanente Haarfärbemittel, Präparate mit selbstoxidierenden Farbstoffen, oder natürliche Haarfärbemittel, wie Henna oder Kamille.

Diese aufgezählten Formulierungen können in verschiedenen Darreichungsformen vorliegen, wie z.B.
- in Form von flüssige Zubereitungen als einer W/O- O/W-, O/W/O-, W/O/W-, PIT (Phasen-Inversions-Temperatur)- und aller Arten von Mikroemulsionen
- in Form eines Gels,
- in Form eines Öls, einer Creme, Milch oder Lotion,
- in Form eines Pulvers, eines Lacks, einer Tablette oder Make-Ups,
- in Form eines Stiftes,
- in Form eines Sprays (Spray mit Treibgas oder Pumpspray) oder eines Aerosols,
- in Form eines Schaumes, oder
- in Form einer Paste.

Die kosmetischen Formulierungen zeichnen sich durch exzellenten Schutz der menschlichen Haut gegen den schädigenden Einfluss von Sonnenlicht aus.

In den folgenden Beispielen beziehen sich die Prozentsätze auf das Gewicht. Die Mengen beziehen sich auf die Reinsubstanz.

### Herstellungsbeispiele:

Beispiel 1: 10 g 2-(1.3.3.-Trimethyl-indolin-2-ylen)-acetaldehyd (= Fischerbase-aldehyd; technische Qualität) werden in 250 ml Petrolether (Siedebereich 80-110°C) unter Rückfluss gelöst. Mit 1 g Filterhilfsmittel wird klärfiltriert und unter Rühren langsam abgekühlt. Nach 16 Stunden wird bei 25°C filtriert und getrocknet. Man erhält 6 g reine, leicht gelb gefärbte Kristalle der Formel 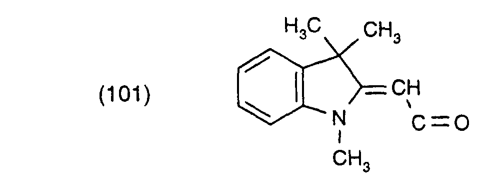 mit einem Schmelzpunkt von 105-106°C.
ε = 34520 l/(mol/cm) in Ethanol bei λ_{max.} = 341 nm:
Photostabilität t_{½} = 208 Stunden

Beispiel 2: Man löst 5 g 2-(1.3.3.-Trimethyl-indolin-2-ylen)-acetaldehyd (= Fischerbase-aldehyd) in 50 ml Toluol. Dann gibt man 5 Tropfen Piperidin und 0,5 g 100%-ige Essigsäure zu, versetzt mit 5 g Cyanessigsäure-2-ethylester und heizt bei 100 bis 110°C unter Rückfluss. Man hält diese Temperatur während 3 Std. und kreist dabei das sich bildende Wasser mit einem Abscheider aus. Nun wird das Toluol grösstenteils ausdestilliert und der Rückstand in 80 ml Petrolether (Siedebereich 80-110°C) heiss gelöst und klärfiltriert. Durch langsames Abkühlen kristallisiert die Verbindung der Formel 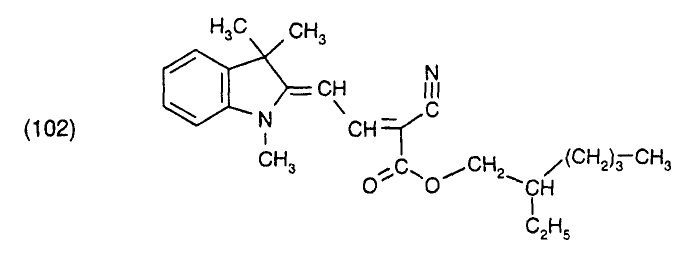 aus und wird bei 25 °C abfiltriert. Nach dem Trocknen erhält man 7,1 g gelbe Kristalle mit einem Schmelzpunkt von 75 - 77°C.
ε = 62081 in Ethanol l/(mol/cm) bei λₘₐₓ = 434nm ,
Elementaranalyse für C₂₄ H₃₂ N₂ O₂ [%]:

| | C | H | N | O |
|---|---|---|---|---|
| berechnet | 75,75 | 8,48 | 7,37 | 8,41 |
| gefunden | 75,7 | 8,5 | 7,4 | 8,6 |

Beispiel 3: Man löst 17.3 g 1.3.3.-Trimethyl-2-methylen-indolin (= Fischersche Base) in 30 ml Methylenchlorid und tropft in einer halben Stunde bei 20 - 32°C 14 g Benzoylchlorid zu. Man erwärmt die Reaktionsmasse auf 45°C, hält diese Temperatur während einer halben Stunde, kühlt auf 25°C ab und filtriert vom ausgefallenen Edukt-Hydrochlorid. Das rote Filtrat wird zur Trockene eingeengt und aus dem Gemisch vom 30 ml Aceton/3 ml Wasser umkristallisiert. Man erhält so 8.7 g der Verbindung der Formel 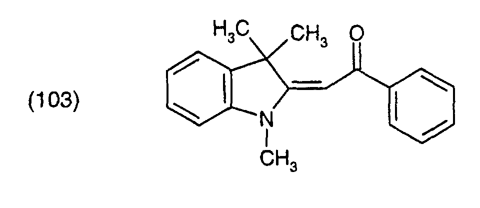 als fahlgelbe Kristalle mit einem Schmelzpunkt von 133-134°C.
ε = 29427 in Dioxan l/(mol/cm) bei λₘₐₓ = 377 nm.

### Applikationsbeispiele:

### Beispiel 4: Herstellung einer O/W-Lotion

| INCl-Bezeichnung | % |
|---|---|
| Polyglyceryl-3 Methylglucose Distearate | 3,0 |
| Decyl Oleate | 7,2 |
| Isopropyl Palmitate | 7,0 |
| Caprylic/Capric Triglyceride | 8,4 |
| Verbindung der Formel (103) | 4,0 |
| Octyl Methoxycinnamate | 5,0 |
| Glycerol | 3,0 |
| Phenoxyethanol & (Methyl, Ethyl, Propyl, Butyl)Parabens | 0,5 |
| deionisiertes Wasser | 60,9 |
| Carbomer | 0,2 |
| Isopropyl Palmitate | 0,8 |
| NaOH (10%) | nach Bedarf |

Die Ölphase und die Wasserphase werden getrennt auf 75-80°C erwärmt und vorsichtig zusammengegeben. Danach folgen intensive Homogenisierung und Abkühlen auf Raumtemperatur unter leichten Rühren.

Mit dem Optometrics SPF-290 Analyzer (2 µl/cm² auf Transpore-Film) wird ein *in vitro*-SPF von 15 bestimmt. Der Australische Standard für den UVA-Schutz (Australian / New Zealand Standard, 15 / NZS 2604: 1993) wird erfüllt.

### Beispiel 5:. Herstellung einer O/W-Emulsion

| | INCl-Bezeichnung | % |
|---|---|---|
| A | Polyglyceryl-3 Methylglucose Distearate | 2,5 |
| | Decyl Oleate | 7,7 |
| | Isopropyl Palmitate | 7,0 |
| | Vitamin E Acetate | 1,5 |
| | Caprylic/Capric Triglyceride | 9,5 |
| | Bis-Octylphenol Methoxyphenyl Triazine | 3,0 |
| | Verbindung der Formel (101) | 3,5 |
| | | |
| B | Glycerol | 3,0 |
| | Phenoxyethanol & (Methyl, Ethyl, Propyl, Butyl) Parabens | 0,5 |
| | deionisiertes Wasser | 64,3 |
| | | |
| C | Carbomer | 0,2 |
| | Isopropyl Palmitate | 0,8 |
| | | |
| E | NaOH (10%) | nach Bedarf |

Phase C wird mit Phase B vereinigt und dann die Phasen A und B separat auf 75-80°C erwärmt, vorsichtig zusammengegeben und homogenisiert. Nach Abkühlen unter langsamen Rühren wird mit E ein pH von 7,0 eingestellt.

Mit dem Optometrics SPF-290 Analyzer (2 µl/cm² auf Transpore-Film) wird ein *in vitro*-SPF von 18 bestimmt. Der Australische Standard für den UVA-Schutz (Australian/New Zealand Standard, 15 / NZS 2604: 1993) wird erfüllt.

### Beispiel 6: Herstellung einer W/O Emulsion

| INCl-Bezeichnung | % w/w |
|---|---|
| PEG-30 Dipolyhydroxy-Stearate | 3,50 |
| PEG-22/Dodecyl Glycol Copolymer | 1,50 |
| Microcrystalline Wax | 1,00 |
| Hydrogenated Castor Oil | 1,00 |
| Magnesium Stearate | 1,00 |
| Octyl Stearate | 15,00 |
| Coco Glycerides | 2,00 |
| Mineral Oil | 3,00 |
| Phenoxyethanols and (Methyl, Ethyl, Propyl, Butyl)Parabens | 1,00 |
| Octyl Methoxycinnamate | 5,00 |
| Dimethicone | 0,10 |
| deionisiertes Wasser | 49,90 |
| Allantoin | 0,10 |
| Magnesium Sulfate | 1,00 |
| Verbindung der Formel (102) | 5,00 |
| Propylene Glycole | 4,00 |
| Methylene Bis-Benzotriazolyl Tetrametylbutylphenol(pH 5,5) | 6,00 |

Die Ölphase und die Wasserphase werden getrennt auf 75-80°C erwärmt und vorsichtig zusammengegeben. Danach folgen intensive Homogenisierung und Abkühlung auf Raumtemperatur unter leichten Rühren. Zur erhaltenen Emulsion wird Methylene Bis-Benzotriazolyl Tetrametylbutylphenol unter Rühren dazugemischt.

Mit dem Optometrics SPF-290 Analyzer (2 µl/cm² auf Transpore-Film) wird ein *in vitro*-SPF von 24 bestimmt.

### Beispiel 7: Herstellung einer W/O-Emulsion

| INCl-Bezeichnung | Formulierung (A) % | Formulierung (B) % |
|---|---|---|
| Methoxy PEG-22/Dodecyl Glycol Copolymer | 3,00 | 3,00 |
| PEG-22/Dodecyl Glycol Copolymer | 3,00 | 3,00 |
| Hydroxyoctacosanyl Hydroxystearat | 3,00 | 3,00 |
| Octyl Stearate | 15,00 | 15,00 |
| Coco Glycerides | 2,00 | 2,00 |
| Mineral Oil | 3,00 | 3,00 |
| Phenoxyethanols and (Methyl, Ethyl, Propyl, Butyl)Parabens | 1,00 | 1,00 |
| Octyl Methoxycinnamate | 4,00 | 5,00 |
| Dimethicone | 0,20 | 0,10 |
| deionisiertes Wasser | 47,70 | 43,80 |
| Allantoin | 0,10 | 0,10 |
| Verbindung der Formel (102) | 5,00 | 4,00 |
| Magnesium Sulfate | 1,00 | 1,00 |
| Propylene Glycole | 4,00 | 4,00 |
| Methylene Bis-Benzotriazolyl Tetrametylbutylphenol (pH 5,5) (50%ige Suspension) | 8,00 | 12,00 |

Die Ölphase und die Wasserphase werden getrennt auf 75-80°C erwärmt und vorsichtig zusammengegeben. Danach folgen intensive Homogenisierung und Abkühlung auf Raumtemperatur unter leichten Rühren. Zur erhaltenen Emulsion wird Methylene Bis-Benzotriazolyl Tetrametylbutylphenol unter Rühren zugemischt.

Mit dem Optometrics SPF-290 Analyzer (2 µl/cm² auf Transpore-Film) werden *in vitro*-SPF-Werte von 20 (A) und 28 (B) bestimmt. Der Australische Standard für den UVA-Schutz (Australian / New Zealand Standard, 15 / NZS 2604: 1993) wird erfüllt.

## Patentansprüche

1. Nichttherapeutische Verwendung der Indolinderivate der Formel 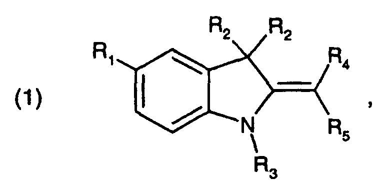 worin
R₁ Wasserstoff; C₁-C₅-Alkyl; C₁-C₁₈-Alkoxy; oder Halogen;
R₂ C₁-C₈-Alkyl; C₅-C₇-Cycloalkyl; C₆-C₁₀-Aryl;
R₃ C₁-C₁₈-Alkyl; oder ein Rest der Formel (1a) 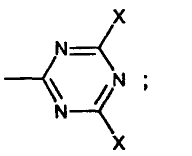
R₄ Wasserstoff; oder ein Rest der Formel (1b) 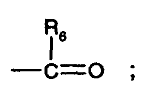
R₅ einen Rest der Formel (1c) 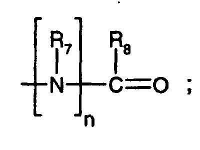 C₁-C₁₈-Alkoxy; oder einen Rest der Formel (1d) 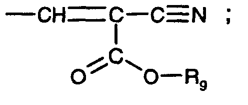
R₆ und R₇ unabhängig voneinander Wasserstoff; oder C₁-C₅-Alkyl;
R₈ Wasserstoff; C₁-C₅-Alkyl; C₅-C₇-Cycloalkyl; Phenyl; Phenyl-C₁-C₃-Alkyl;
R₉ C₁-C₁₈-Alkyl;
X Hal; ein Rest der Formel (1e) 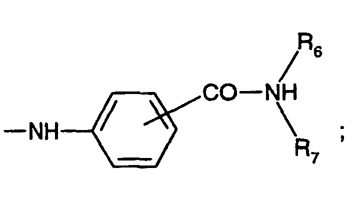 oder(1f) 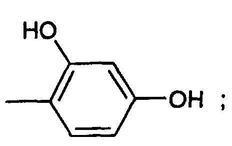 und
n 0; oder 1;
bedeuten, als Lichtschutzmittel.

2. Verwendung nach Anspruch 1, **dadurch gekennzeichnet, dass** in Formel (1)
R₅ einen Rest der Formel (1g) 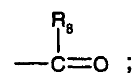 und
R₈ Wasserstoff; C₁-C₅-Alkyl; oder Phenyl;
bedeuten.

3. Verwendung nach Anspruch 2, **dadurch gekennzeichnet, dass**
R₈ Wasserstoff oder Methyl bedeutet.

4. Verwendung nach Anspruch 1, **dadurch gekennzeichnet, dass**
R₅ C₁-C₁₈-Alkoxy;
bedeutet.

5. Verwendung nach Anspruch 4, **dadurch gekennzeichnet, dass**
R₅ einen Rest der Formel (1h) 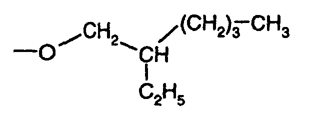
bedeutet.

6. Verwendung nach Anspruch 1, **dadurch gekennzeichnet, dass**
R₅ einen Rest der Formel (1i) 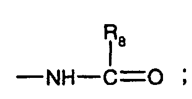 und
R₈ Wasserstoff; oder Methyl bedeutet.

7. Verwendung nach einem der Ansprüche 1 bis 6, **dadurch gekennzeichnet, dass**
R₃ C₃-C₁₈-iso-Alkyl
bedeutet.

8. Nichttherapeutische Verwendung der Verbindungen der Formel (1) zum Schützen von menschlichen und tierischen Haaren und der Haut vor der schädigenden Einwirkung von UV-Strahlung.

9. Kosmetische Zusammensetzung, enthaltend mindestens eine oder mehrere Verbindungen der Formel (1) nach Anspruch 1 mit kosmetisch verträglichen Träger- oder Hilfsstoffen.

10. Zusammensetzung nach Anspruch 9, **dadurch gekennzeichnet, dass** es weitere UV-Schutzstoffe enthält.

11. Zusammensetzung nach Anspruch 9 oder 10, **dadurch gekennzeichnet, dass** es als weitere UV- Schutzstoffe Triazine, Oxanilide, Triazole, Vinylgruppen enthaltende Amide oder Zimtsäureamide enthält.

## Claims

1. Non-therapeutic use of an indoline derivative of formula 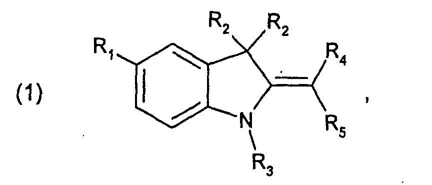 wherein
R₁ is hydrogen; C₁-C₅alkyl; C₁-C₁₈alkoxy; or halogen;
R₂ is C₁-C₈alkyl; C₅-C₇cycloalkyl; C₆-C₁₀aryl;
R₃ is C₁-C₁₈alkyl; or a radical of formula (1a) 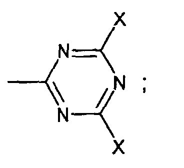
R₄ is hydrogen; or a radical of formula (1b) 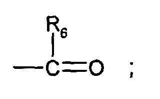
R₅ is a radical of formula (1c) 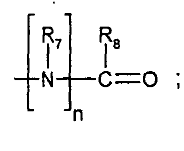 C₁-C₁₈alkoxy; or a radical of formula (1d) 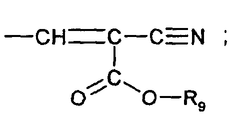
R₆ and R₇ are each independently of the other hydrogen; or C₁-C₅alkyl;
R₈ is hydrogen; C₁-C₅alkyl; C₅-C₇cycloalkyl; phenyl; phenyl-C₁-C₃alkyl;
R₉ is C₁-C₁₈alkyl;
X is halogen; a radical of formula (1e) 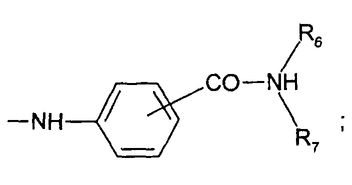 or (1f) 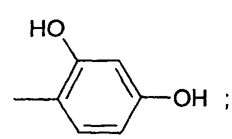 and
n is 0; or 1,
as a light-protective agent.

2. Use according to claim 1, wherein, in formula (1),
R₅ is a radical of formula (1g) 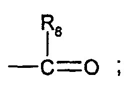 and
R₈ is hydrogen; C₁-C₅alkyl; or phenyl.

3. Use according to claim 2, wherein
R₈ is hydrogen or methyl.

4. Use according to claim 1, wherein
R₅ is C₁-C₁₈alkoxy.

5. Use according to claim 4, wherein
R₅ is a radical of formula (1h)

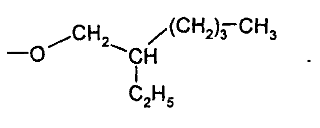

6. Use according to claim 1, wherein
R₅ is a radical of formula (1i) 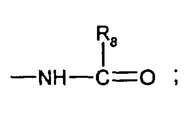 and
R₈ is hydrogen; or methyl.

7. Use according to any one of claims 1 to 6, wherein
R₃ is C₃-C₁₈isoalkyl.

8. Non-therapeutic use of a compound of formula (1) in protecting human and animal hair and the skin against the damaging action of UV radiation.

9. A cosmetic composition comprising at least one or more compounds of formula (1) according to claim 1 together with cosmetically tolerable carriers or adjuvants.

10. A composition according to claim 9, which comprises further UV-protective substances.

11. A composition according to either claim 9 or claim 10, which comprises triazines, oxanilides, triazoles, vinyl-group-containing amides or cinnamic acid amides as further UV-protective substances.

## Revendications

1. Utilisation non thérapeutique des dérivés d'indoline de formule 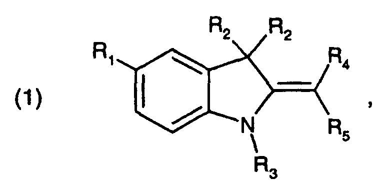 dans laquelle
R₁ représente l'hydrogène, un reste alkyle en C₁-C₅ ou alcoxy en C₁-C₁₈ ou un halogène;
R₂ représente un reste alkyle en C₁-C₈, cycloalkyle en C₅-C₇, aryle en C₆-C₁₀;
R₃ représente un reste alkyle en C₁-C₁₈; ou un reste de formule (1a) 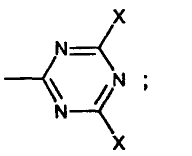
R. représente l'hydrogène; ou un reste de formule (1b) 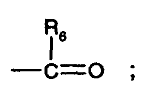
R₅ représente un reste de formule (1c) 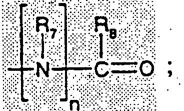 un reste alcoxy en C₁-C₁₈; ou un reste de formule (1d) 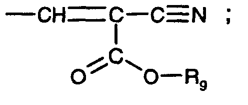
R₆ et R₇ représentent indépendamment l'un de l'autre l'hydrogène ou un reste alkyle en C₁-C₅;
R₈ représente l'hydrogène ou un reste alkyle en C₁-C₅, cycloalkyle en C₅-C₇, phényle, phényle-(alkyle en C₁-C₃);
R₉ représente un reste alkyle en C₁-C₁₈;
X représente Hal; un reste de formule (1e) 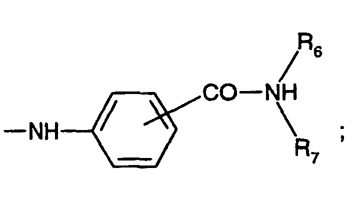 ou (1f) 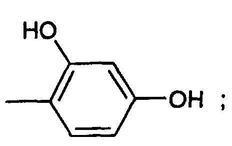 et
n est 0 ou 1,
en tant qu'agents de protection contre la lumière.

2. Utilisation selon la revendication 1, **caractérisée en ce que**, dans la formule (1)
R₅ représente un reste de formule (1g) 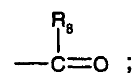 et
R₈ représente l'hydrogène ou un reste alkyle en C₁-C₅ ou phényle.

3. Utilisation selon la revendication 2, **caractérisée en ce que** R₈ représente l'hydrogène ou un reste méthyle.

4. Utilisation selon la revendication 1, **caractérisée en ce que** R₅ représente un reste alcoxy en C₁-C₁₈.

5. Utilisation selon la revendication 4, **caractérisée en ce que** R₅ représente un reste de formule (1h)

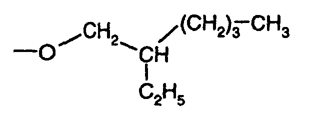

6. Utilisation selon la revendication 1, **caractérisée en ce que**
R₅ représente un reste de formule (1i) 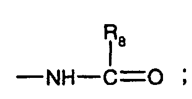 et
R₈ représente l'hydrogène ou un reste méthyle.

7. Utilisation selon l'une des revendications 1 à 6, **caractérisée en ce que** R₃ représente un reste isoalkyle en C₃-C₁₈.

8. Utilisation non thérapeutique des composés de formule (1) pour la protection des cheveux ou des poils et de la peau d'êtres humains et d'animaux contre les effets nocifs du rayonnement UV.

9. Composition cosmétique contenant au moins un ou plusieurs composés de formule (1) selon la revendication 1 avec des supports ou des agents auxiliaires cosmétiquement acceptables.

10. Composition selon la revendication 9, **caractérisée en ce qu'**elle contient d'autres agents protecteurs contre les UV.

11. Composition selon la revendication 9 ou 10, **caractérisée en ce qu'**elle contient comme autres agents protecteurs contre les UV des triazines, des oxanilides, des triazoles, des amides contenant des groupes vinyle ou des amides d'acide cinnamique.
